Europäisches Patentamt

European Patent Office

Office européen des brevets

(11) Veröffentlichungsnummer: **0 264 126**
**B1**

(12) **EUROPÄISCHE PATENTSCHRIFT**

(45) Veröffentlichungstag der Patentschrift: **30.01.91**

(51) Int. Cl.$^5$: **C 07 D 201/16**

(21) Anmeldenummer: **87115062.9**

(22) Anmeldetag: **15.10.87**

(54) **Verfahren zur Neutralisation von Reaktionsgemischen, die durch Beckmann'sche Umlagerung von Cyclohexanonoxim erhalten worden sind.**

(30) Priorität: **17.10.86 DE 3635363**

(43) Veröffentlichungstag der Anmeldung:
**20.04.88 Patentblatt 88/16**

(45) Bekanntmachung des Hinweises auf die
Patenterteilung:
**30.01.91 Patentblatt 91/05**

(84) Benannte Vertragsstaaten:
**BE CH DE ES FR GB IT LI NL**

(56) Entgegenhaltungen:
**DE-A-2 651 195**
**GB-A- 996 322**
**US-A-2 605 261**

(73) Patentinhaber: **BASF Aktiengesellschaft**
**Carl-Bosch-Strasse 38**
**D-6700 Ludwigshafen (DE)**

(72) Erfinder: **Brand, Uwe**
**Rheingoldstrasse 12**
**D-6840 Lampertheim (DE)**
Erfinder: **Schmitz, Ruediger**
**Roemerstrasse 2**
**D-6715 Lambsheim (DE)**
Erfinder: **Deuker, Ernst, Dr.**
**Westring 31**
**D-6718 Gruenstadt (DE)**
Erfinder: **Fuchs, Hugo, Dr.**
**Egellstrasse 28**
**D-6700 Ludwigshafen (DE)**

EP 0 264 126 B1

**Beschreibung**

Bei der Beckmann'schen Umlagerung von Cyclohexanonoxim mit Schwefelsäure oder Oleum erhält man ein Reaktionsgemisch, das im wesentlichen aus rohem Caprolactam (Rohlactam) und Schwefelsäure besteht. Solche Reaktionsgemische werden mit Ammoniak neutralisiert und Rohlactam und festes Ammonsulfat abgetrennt. In der DE-AS 26 51 195 wird ein Verfahren beschrieben, bei dem das Reaktionsgemisch aus der Beckmann'schen Umlagerung durch Neutralisiation mit Ammoniak bei erhöhter Temperatur unter Zusatz von zurückgeführter Ammonsulfatmutterlauge, wobei die Konzentration so gewählt wird, daß während der Neutralisation kein festes Ammonsulfat ausfällt. Abtrennen des Rohcaprolactams von der Ammonsulfatlösung, Kristallisation von Ammonsulfat durch Eindampfen der Ammonsulfatlösung unter vermindertem Druck, Abtrennen des kristallisierten Ammonsulfats und Rückführung der Mutterlauge in die Neutralisationsstufe aufgearbeitet wird. Die Neutralisation kann hierbei mit gasförmigem Ammoniak oder Ammoniak in wäßriger Lösung oder zusammen mit Ammonsulfatmutterlauge erfolgen, wobei die Vereinigung von Ammoniak und Ammonsulfatlösung vor der Neutralisationsstufe aber auch unmittelbar in der Neutralisationsstufe erfolgen kann.

Nach dem in der US-A-2 605 261 beschriebenen Verfahren wird das Umlagerungsgemisch aus der Beckmann'schen Umlagerung zunächst mit Ammonsulfatmutterlauge in einer solchen Menge gemischt, daß kein festes Ammoniumsulfat ausfällt und dann das Gemisch mit Ammoniak oder wäßrigem Ammoniak neutralisiert. Bei der Neutralisation soll eine innige Durchmischung erfolgen.

Das in der GB-A-996 322 beschriebene Verfahren sieht vor, daß man in ein Mischgefäß gleichzeitig das Umlagerungsgemisch aus der Beckmann'schen Umlagerung, Ammonsulfatmutterlauge und Ammoniak oder wäßriges Ammoniak einbringt und dort unter gutem Durchmischen für sofortige Neutralisation sorgt.

Bei der Neutralisation unter Zuführung von gasförmigem Ammoniak durch eine Vielzahl von Düsenöffnungen hat es sich herausgestellt, daß diese Düsenöffnungen innerhalb von kurzer Zeit verstopfen und eine gleichmäßige Zuführung von Ammoniak in das zu neutralisierende Medium und somit dessen zufriedenstellende Neutralisation nicht gewährleistet ist.

Es war deshalb die technische Aufgabe gestellt, ein Verfahren zur Neutralisation von Reaktionsgemischen aus der Beckmann'schen Umlagerung zur Verfügung zu stellen, bei dem die Zuführung von gasförmigem Ammoniak bei der Neutralisation ohne Schwierigkeiten verläuft und eine gleichmäßige Neutralisation des Reaktionsgemisches gewährleistet wird.

Diese Aufgabe wird gelöst in einem Verfahren zur Neutralisation von Reaktionsgemischen, die durch Beckmann'sche Umlagerung von Cyclohexanonoxim mit Schwefelsäure oder Oleum erhalten worden sind, das folgende Schritte umfaßt

a) Mischen des Reaktionsgemisches mit zurückgeführter Ammonsulfatmutterlauge, deren Konzentration so gewählt ist, daß bei der Neutralisation kein festes Ammonsulfat ausfällt,

b) Neutralisation durch Zuführen von gasförmigem Ammoniak bis pH 3 bis 6 bei einer Temperatur von 80 bis 115°C unter autogenem Druck,

c) Abtrennen von Rohlactam von der wäßrigen Ammonsulfatlösung

d) Eindampfen der Ammonsulfatlösung unter vermindertem Druck von 200 bis 800 mbar unter Abkühlung auf 72 bis 102°C und Abtrennen des kristallisierten Ammonsulfats von der Ammonsulfatmutterlauge

e) Zurückführen der Ammonsulfatmutterlauge in Stufe a)

dadurch gekennzeichnet, daß man gasförmiges Ammoniak, das wasser oder eine wäßrige Ammonsulfatlösung in flüssiger Form in feiner Verteilung enthält, durch eine Vielzahl von Düsenöffnungen der Lösung von Reaktionsgemisch in Ammonsulfatmutterlauge zuführt.

Das neue Verfahren hat den Vorteil, daß keine Verstopfungen bei der Zuführung von gasförmigem Ammoniak auftreten und zudem eine gleichmäßige Neutralisation der Lösung von Reaktionsgemisch in der Ammonsulfatmutterlauge gewährleistet wird.

Man geht von Reaktionsgemischen aus, die durch Beckmann'sche Umlagerung von Cyclohexanonoxim mit Schwefelsäure oder Oleum erhalten worden sind. Typische Gemische enthalten 40 bis 50 Gew.% Caprolactam und 50 bis 60 Gew.% Schwefelsäure. In der Stufe a) wird zunächst das Reaktionsgemisch mit zurückgeführter Ammonsulfatmutterlauge, deren Konzentration so gewählt ist, daß bei der Neutralisation kein festes Ammonsulfat ausfällt, gemischt. Die Konzentration der verwendeten Ammonsulfatmutterlauge wird deshalb höchstens so groß gewählt, daß sich nach der Neutralisation eine nahezu gesättigte Lösung bildet. wenn man bei der Neutralisation eine Temperaturerhöhung von 10 bis 40°C vorsieht, so wird vorteilhaft die 5 bis 50-fache Menge an Ammonsulfatmutterlauge, bezogen auf das umlagerungsgemisch, verwendet. Eine geeignete Konzentration erzielt man vorteilhaft, wenn man die Ammonsulfatmutterlauge vorher verdünnt, insbesondere im Verhältnis Mutterlauge zu wasser in einem Verhältnis von 5 bis 65:1. Eine geeignete Ammonsulfatmutterlauge hat z.B. eine Konzentration von 35 bis 45 Gew.%.

In der Stufe b) wird die Neutralisation durch Zuführen von gasförmigem Ammoniak bei erhöhter Temperatur durchgeführt. Gasförmiges Ammoniak wird durch eine Vielzahl von Düsenöffnungen, z.B. 50 bis 2000 Düsenöffnungen je m² mit einem Durchmesser von 1 bis 5 mm der Lösung von Reaktionsgemisch in Ammonsulfatmutterlauge zugeführt. Die Zuführung erfolgt zweckmäßig in einem Mischrohr, um eine rasche Durchmischung und somit Neutralisation zu gewährleisten. In der Neutralisationszone hält man einen pH-Wert von 3 bis 6, insbesondere von 4,5 bis 5,0 ein. Die Neutralisation wird bei einer Temperatur von 80 bis 115°C durchgeführt. Der Druck entspricht dann dem autogenen Druck bei der jeweiligen Neutralisationstemperatur und Zusammensetzung des neutralisierten Gemisches.

Erfindungsgemäß wird dem gasförmigen Ammoniak vor der Zuführung Wasser oder eine wäßrige Ammoniumsulfatlösung, z.B. bis zu einer Konzentration von 35 Gew.% Ammoniumsulfat, bevorzugt 20 bis 30 Gew.% in flüssiger Form in feiner Verteilung zugesetzt. Dies erfolgt zweckmäßig durch Einsprühen der genannten Medien in den Ammoniakgasstrom mit einer Sprühdüse, wobei vorteilhaft eine mittlere Tropfengröße von 0,2 bis 2,0 mm erzeugt wird. Vorteilhaft wendet man je m³ gasförmigem Ammoniak von etwa 25°C mindestens 0,05 l Wasser oder mindestens 0,1 l wäßrige Ammonsulfatlösung an. Besonders bewährt haben sich 0,05 bis 0,5 l, insbesondere 0,05 bis 0,25 l Wasser je m³ gasförmiges Ammoniak oder 0,1 bis 1,0 l, insbesondere 0,1 bis 0,5 l Ammonsulfatlösung je m³ gasförmigem Ammoniak.

Das neutralisierte Gemisch wird anschließend in der Stufe c) in eine Trennzone geleitet, wo die beiden flüssigen Phasen, das Rohlactam und die wäßrige Ammonsulfatlösung getrennt werden. Diese Trennung kann beispielsweise nach Scheidung in zwei Schichten durch einfaches Abziehen (Dekantieren) oder unter Verwendung von Separatoren erfolgen. Es versteht sich, daß der autogene Druck zweckmäßig aufrecht erhalten wird.

Die leichte Phase enthält das wasserhaltige Rohlactam, die vorteilhaft gekühlt wird, um Nebenproduktbildung zu vermeiden. Diese wird dann mit Benzol extrahiert und aus der benzolischen Lösung Caprolactam gewonnen.

Die in der Trennzone abgeschiedene Ammonsulfatlösung wird zweckmäßig ohne diese abzukühlen, einer Verdampferkristallisierstufe d) zugeführt, wo unter vermindertem Druck und Abtrennen des kristallisierten Ammonsulfats eine Ammonsulfatmutterlauge erhalten wird. Das Eindampfen erfolgt unter einem Druck von 200 bis 800 mbar, Hierbei wird Wasser verdampft, bis die Ammonsulfatlösung eine Temperatur von 72 bis 101°C erreicht hat. Durch Übersättigung kristallisiert hierbei Ammonsulfat aus. Es versteht sich, daß man nicht vollständig, sondern immer nur so weit eindampft, daß soviel Ammonsulfat auskristallisiert, wie bei der Neutralisation entsteht. Danach wird auch die zu verdampfende Menge wasser bemessen. Die erhaltene Salzmaische wird einer Trennvorrichtung zugeführt, z.B. einem Filter oder einer Zentrifuge.

Man erhält so eine Ammonsulfatmutterlauge, die entsprechend Stufe e) wieder in die Stufe a) zurückgeführt wird. Es versteht sich, daß der Ammonsulfatmutterlauge zweckmäßig so viel Wasser zugesetzt wird, wie der verdampften Wassermenge entspricht. Vorteilhaft verwendet man hierfür das Kondensat, der aus dem Verdampfer bzw. Kristaller abgezogenen Brüden. Es soll auch die Wassermenge ersetzt werden, die mit dem Lactam abgetrennt wird. Auf jeden Fall muß eine so große Menge an Wasser zugegeben werden, daß während der Neutralisiation Ammonsulfat nicht ausfällt. Die Mindestmenge an Verdünnungswasser hängt wegen der temperaturabhängigen Löslichkeit des Ammonsulfats, von der bei der Neutralisation erreichten Temperatur sowie von der angestrebten Konzentration der Ammonsulfatlösung ab. Im allgemeinen sind auf ein Teil zu erwartendes Ammoniumsulfat 1 bis 1,5 Teile Verdünnungswasser ausreichend.

Das Verfahren nach der Erfindung sei an folgenden Beispielen veranschaulicht. Die angegebenen Teile verhalten sich zu den Volumenteilen wie kg zu Liter.

Beispiel 1

12,4 Teile eines schwefelsauren Umlagerungsgemisches von Cyclohexanonoxim (mit einer Zusammensetzung von 42,7 Gew.% Caprolactam und 57,3 Gew.% Schwefelsäure) werden in einem Kreislauf zunächst mit 200 Teilen/Stunde einer Ammoniumsulfatlösung mit einer Konzentration von 42,5 Gew.% gemischt und durch ein Mischrohr geleitet, in dem über 1200 Düsen von 4 mm Durchmesser 3220 Volumenteile gasförmiges Ammoniak zugeführt werden. Im gasförmigen Ammoniak werden mit einer Sprühdüse bei 2 bar Differenz-Druck für jeden Kubikmeter Ammoniakgas 0,13 l Wasser zugeführt, wodurch sich Tropfen von einer mittleren Tropfengröße von 1 mm Durchmesser bilden. In der Neutralisationszone erhöht sich die Temperatur von 90°C auf 100°C und es stellt sich ein pH-Wert von 4,5 ein. Der autogene Druck beträgt 1 bar. Das Neutralisationsgemisch wird dann in einen Trennbehälter geleitet, in dem stündlich 3,80 Teile wasserhaltiges Rohcaprolactam mit einem Gehalt von etwa 68 Gew.% Caprolactam als leichte Phase und 209,5 Teile konzentrierte Ammonsulfatlösung als schwere Phase voneinander geschieden werden. Die konzentrierte Ammonsulfatlösung wird in einem Kristallererdampfer geleitet und unter einem Druck von 540 torr stündlich 4,8 Teile Wasser verdampft. Hierbei kühlt sich die Lösung auf 90°C ab, wobei Übersättigung und Kristallisation eintritt. Aus der erhaltenen Salzmaische werden in einer Zentrifuge 4,7 Teile Ammonsulfat abgetrennt. Es verbleiben stündlich 200 Teile Ammonsulfatmutterlauge, die wieder mit 4,8 Teilen Wasser verdünnt und in die Stufe a) zurückgeführt werden. Nach einer Betriebsdauer con 30 Tagen konnte noch keine Verstopfung der Zuführungsöffnungen für das gasförmige Ammoniak beobachtet werden.

Beispiel 2

Man verfährt wie in Beispiel 1 beschrieben, fügt dem gasförmigen Ammoniak mittels einer Sprühdüse bei 2 bar Differenzdruck je m³ Ammoniakgas 0,26 l 23 gew.%ige wäßrige Ammonsulfatlösung zu. Während eines Betriebes von 30 Tagen konnte

3

keine Verstopfung der Zuführungsöffnungen für das gasförmige Ammoniak beobachtet werden.

Vergleichsbeispiel 1

Man verfährt wie in Beispiel 1 beschrieben, und führt gasförmiges Ammoniak jedoch ohne weitere Zusätze durch die Düsenöffnungen zu. Bereits nach einigen Minuten beginnt der Druck in der Zuführungsleitung für gasförmiges Ammoniak anzusteigen und nach kurzer Zeit muß die Anlage außer Betrieb genommen werden, da die Zuführungsöffnungen für die Ammoniakeinspeisung mit auskristallisiertem Ammonsulfat verstopft sind.

Vergleichsbeispiel 2

Man verfährt wie in Beispiel 1 beschrieben, setzt dem Ammoniakgasstrom jedoch je m³ Ammoniak 200 g Wasser als Dampf von 120°C zu. Die Anlage mußte nach 0,5 Stunden außer Betrieb genommen werden, da die Zuführungsöffnungen für den gasförmigen Ammoniak durch auskristallisiertes Ammonsulfat verstopft waren.

**Patentansprüche**

1. Verfahren zur Neutralisation von Reaktionsgemischen, die durch Beckmann'sche Umlagerung von Cyclohexanonoxim mit Schwefelsäure oder Oleum erhalten worden sind, welches folgende Schritte umfaßt
   a) Mischen des Reaktionsgemisches mit zurückgeführter Ammonsulfatmutterlauge, deren Konzentration so gewählt ist, daß bei der Neutralisation kein festes Ammonsulfat ausfällt,
   b) Neutralisation durch Zuführen von gasförmigem Ammoniak bis pH 3 bis 6 bei einer Temperatur von 80 bis 115°C unter autogenem Druck
   c) Abtrennen von Rohlactam von der wäßrigen Ammonsulfatlösung
   d) Eindampfen der Ammonsulfatlösung unter vermindertem Druck von 200 bis 800 mbar unter Abkühlung auf 72 bis 101°C und Abtrennen des kristallisierten Ammonsulfats von der Ammonsulfatmutterlauge
   e) Zurückführen der Ammonsulfatmutterlauge in Stufe a) dadurch gekennzeichnet, daß man gasförmiges Ammoniak, das Wasser oder eine wäßrige Ammonsulfatlösung in flüssiger Form in feiner Verteilung enthält, durch eine Vielzahl von Düsenöffnungen der Lösung des Reaktionsgemischs in der Ammonsulfatmutterlauge zuführt.
   2. Verfahren nach Anspruch 1, dadurch gekennzeichnet, daß man in Stufe a) das Reaktionsgemisch mit der 5- bis 50-fachen Menge an zurückgeführter Ammonsulfatmutterlauge mischt.
   3. Verfahren nach den Ansprüchen 1 und 2, dadurch gekennzeichnet, daß man dem gasförmigen Ammoniak je m³ mindestens 0,05 1 Wasser oder mindestens 0,1 1 wäßrige Ammonsulfatlösung in flüssiger Form in feiner Verteilung zusetzt.
   4. Verfahren nach den Ansprüchen 1 bis 3, dadurch gekennzeichnet, daß das gasförmige Ammoniak Wasser oder wäßrige Ammonsulfatlösung in einer Tröpfchengröße von 0,2 bis 2,0 mm enthält.
   5. Verfahren nach den Ansprüchen 1 bis 4, dadurch gekennzeichnet, daß man der Ammonsulfatmutterlauge vor dem Zurückführen in Stufe a) Wasser im Verhältnis Mutterlauge zu wasser von 5 bis 65:1 zusetzt.

**Revendications**

1. Procédé de neutralisation de mélanges réactionnels qui ont été obtenus par transposition de Beckmann de cyclohexanone-oxime sous l'action d'acide sulfurique ou d'oléum, comprenant les étapes suivantes
   a) mélangeage du mélange réactionnel avec de la liqueurmère de sulfate d'ammonium recyclée, dont la concentration est choisie de telle sorte qu'il ne précipite pas de sulfate d'ammonium solide lors de la neutralisation,
   b) neutralisation par apport d'ammoniac gazeux jusqu'à un pH de 3 à 6, à une température de 80 à 115°C sous la pression autogène,
   c) séparation du lactame brut d'avec la solution aqueuse de sulfate d'ammonium,
   d) évaporation de la solution de sulfate d'ammonium sous une pression réduite de 200 à 800 mbar avec refroidissement à une température de 72 à 101°C et séparation du sulfate d'ammonium cristallisé d'avec la liqueur-mère de sulfate d'ammonium,
   e) recyclage de la liqueur-mère de sulfate d'ammonium dans l'étape a), caractérisé en ce qu'on envoie de l'ammoniac gazeux, qui contient de l'eau ou une solution aqueuse de sulfate d'ammonium sous forme liquide à l'état finement divisé, à travers une multiplicité d'orifices de buse, dans la solution du mélange réactionnel dans la liqueur-mère de sulfate d'ammonium.
   2. Procédé selon la revendication 1, caractérisé en ce que dans l'étape a), le mélange réactionnel est mélangé avec 5 à 50 fois sa quantité de liqueur-mère de sulfate d'ammonium recyclée.
   3. Procédé selon la revendication 1 ou 2, caractérisé en ce qu'on ajoute à l'ammoniac gazeux, par m³, au moins 0,05 1 d'eau ou au moins 0,1 1 de solution aqueuse de sulfate d'ammonium sous forme liquide à l'état finement divisé.
   4. Procédé selon l'une quelconque des revendications 1 à 3, caractérisé en ce que l'ammoniac gazeux contient l'eau ou la solution aqueuse de sulfate d'ammonium sous forme de gouttelettes ayant une grosseur de 0,2 à 2,0 mm.
   5. Procédé selon l'une quelconque des revendications 1 à 4, caractérisé en ce qu'on ajoute à la lessive-mère de sulfate d'ammonium, avant son recyclage dans l'étape a), de l'eau dans un rapport de la liqueur-mère à l'eau de 5 à 65:1.

**Claims**

1. A process for the neutralization of a reaction mixture obtained by Beckmann rearrangement of cyclohexanone oxime with sulfuric acid or oleum, which comprises the following steps:

a) mixing the reaction mixture with recycled ammonium sulfate mother liquor whose concentration is chosen so that no solid ammonium sulfate is precipitated during the neutralization,

b) neutralization by feeding gaseous ammonia up to a pH of from 3 to 6 at from 80 to 115°C under autogenous pressure,

c) separation of crude lactam from the aqueous ammonium sulfate solution,

d) evaporation of the ammonium sulfate solution under reduced pressure of from 200 to 800 mbar with cooling down to from 72 to 101°C and with separation of the crystalline ammonium sulfate from the ammonium sulfate mother liquor and

e) recycling of the ammonium sulfate mother liquor to stage a), wherein gaseous ammonia, which contains water or an aqueous ammonium sulfate solution in finely divided liquid form, is fed through a plurality of nozzle orifices into the solution of the reaction mixture in the ammonium sulfate mother liquor.

2. A process as claimed in claim 1, wherein in stage a), the reaction mixture is mixed with from 5 to 50 times the amount of recycled ammonium sulfate mother liquor.

3. A process as claimed in claims 1 and 2, wherein not less than 0.05 $1/m^3$ of water or not less than 0.1 $1/m^4$ of aqueous ammonium sulfate solution in finely divided liquid form is added to the gaseous ammonia.

4. A process as claimed in any of claims 1 to 3, wherein the gaseous ammonia contains water or aqueous ammonium sulfate solution in a droplet size of from 0.2 to 2.0 mm.

5. A process as claimed in any of claims 1 to 4, wherein water is added to the ammonium sulfate mother liquor in a ratio of mother liquor to water of from 5:1 to 65:1 before the mother liquor is recycled to stage a).